# EUROPEAN PATENT APPLICATION

(11) **EP 1 466 611 A1**
(43) Date of publication of application: **13.10.2004**
(21) Application number: 03076022.7
(22) Date of filing: 08.04.2003
(51) Int. Cl.: A61K 38/06, A61K 38/07, A61K 38/24, A61P 31/00

(54) **Use of HCG derived peptides in the treatment of iatrogenic diseases**

(71) Applicant: Biotempt B.V., 7958 NZ Koekange (NL)
(72) Inventor: The designation of the inventor has not yet been filed

(57) **Abstract**

The invention relates to the field of (veterinary) medicine and to the treatment of subjects (be it man or animal) that suffer from iatrogenic disease, i.e. experience problems or complications resulting from a medical treatment. The invention provides a method for modulating an iatrogenic event in a subject comprising providing said subject with a gene-regulatory peptide or functional analogue thereof.
Furthermore, the invention provides use of an NF-kappaB down-regulating peptide or functional analogue thereof for the production of a pharmaceutical composition for the treatment of an additional pro-inflammatory cytokine response occurring after an iatrogenic event in a subject.

## Description

### Technical field

The current invention relates to the body's innate way of modulation of important physiological processes and builds on insights reported in WO99/59617, WO01/72831 and PCT/NL02/00639.

### Background

In these earlier applications small gene-regulatory peptides are described that are present naturally in pregnant women and are derived from proteolytic breakdown of placental gonadotropins such as human chorionic gonadotropin (hCG) produced during pregnancy. These peptides (in their active state often only at about 4 to 6 amino acids long) were shown to have unsurpassed immunological activity that they exert by regulating expression of genes encoding inflammatory mediators such as cytokines. Surprisingly, it was found that breakdown of hCG provides a cascade of peptides that help maintain a pregnant woman's immunological homeostasis. These peptides are nature's own substances that balance the immune system to assure that the mother stays immunologically sound while her foetus does not get prematurely rejected during pregnancy but instead is safely carried through its time of birth.

Where it was generally thought that the smallest breakdown products of proteins have no specific biological function on their own (except to serve as antigen for the immune system), it now emerges that the body in fact routinely utilises the normal process of proteolytic breakdown of the proteins it produces to generate important gene-regulatory compounds, short peptides that control the expression of the body's own genes. Apparently the body uses a gene-control system ruled by small broken down products of the exact proteins that are encoded by its own genes.

It is long known that during pregnancy the maternal system introduces a status of temporary immuno-modulation which results in suppression of maternal rejection responses directed against the foetus. Paradoxically, during pregnancy, often the mother's resistance to infection is increased and she is found to be better protected against the clinical symptoms of various auto-immune diseases such as rheumatism and multiple sclerosis. The protection of the foetus can thus not be interpreted only as a result of immune suppression. Each of the above three applications have provided insights by which the immunological balance between protection of the mother and protection of the foetus can be understood.

It was shown that certain short breakdown products of hCG (i.e. short peptides which can easily be synthesised, if needed modified, and used as pharmaceutical composition) exert a major regulatory activity on pro- or anti-inflammatory cytokine cascades that are governed by a family of crucial transcription factors, the NFkappaB family which stands central in regulating the expression of genes that shape the body's immune response.

Most of the hCG produced during pregnancy is produced by cells of the placenta, the exact organ where cells and tissues of mother and child most intensely meet and where immuno-modulation is most needed to fight off rejection. Being produced locally, the gene-regulatory peptides which are broken down from hCG in the placenta immediately balance the pro- or anti-inflammatory cytokine cascades found in the no-mans land between mother and child. Being produced by the typical placental cell, the trophoblast, the peptides traverse extracellular space; enter cells of the immune system and exert their immuno-modulatory activity by modulating NFkappaB-mediated expression of cytokine genes, thereby keeping the immunological responses in the placenta at bay.

### Summary of the invention

It is herein postulated that the beneficial effects seen on the occurrence and severity of auto-immune disease in the pregnant woman result from an overspill of the hCG-derived peptides into the body as a whole; however, these effects must not be overestimated, as it is easily understood that the further away from the placenta, the less immuno-modulatory activity aimed at preventing rejection of the foetus will be seen, if only because of a dilution of the placenta-produced peptides throughout the body as a whole. However, the immuno-modulatory and gene-regulatory activity of the peptides should by no means only be thought to occur during pregnancy and in the placenta; man and women alike produce hCG, for example in their pituitaries, and nature certainly utilises the gene-regulatory activities of peptides in a larger whole.

Consequently, a novel therapeutic inroad is provided, using the pharmaceutical potential of gene-regulatory peptides and derivatives thereof. Indeed, evidence of specific up- or down-regulation of NFkappaB driven pro- or anti-inflammatory cytokine cascades that are each, and in concert, directing the body's immune response was found *in silico* in gene-arrays by expression profiling studies, *in vitro* after treatment of immune cells and in vivo in experimental animals treated with gene-regulatory peptides. Also, considering that NFkappaB is a primary effector of disease (A.S. Baldwin, J. Clin. Invest., 2001, 107:3-6), using the hCG derived gene-regulatory peptides offer significant potential for the treatment of a variety of human and animal diseases, thereby tapping the pharmaceutical potential of the exact substances that help balance the mother's immune system such that her pregnancy is safely maintained.

### Detailed description of the invention

The invention in particular relates to the field of (veterinary) medicine and to the treatment of subjects (be it man or animal) that suffer from iatrogenic disease, i.e. experience problems or complications resulting from a medical treatment.

Iatrogenic events that result from activities of for example physicians or surgeons are commonplace in modern medicine and can often not be avoided. Various adverse conditions can occur due to malpractice or neglect, such as wrongly selecting or executing a therapy, misplacing or forgetting to remove surgical utensils during surgery, and the like. However, most therapeutic or surgical interventions, even those well selected and properly executed, may, even beyond their beneficial effects, cause adverse conditions in a patient. Surgeons and clinicians employ various forms of therapy which, although regarded as essential for the patient's well-being, may in them selves have a detrimental effect on host defences. Some of the morbidity (e.g., sepsis) seen in the post-operative period after major surgery may, in part, be due to inhibition of the immune response in the peri-operative period, as a result of the anaesthesia given and the nature of the surgery carried out. Recently, a number of retrospective studies have suggested that blood transfusions may be immuno-inhibitory and detrimental to the survival of patients undergoing surgery for malignant disease. In patients with cancer, increased numbers of malignant cells enter the circulation during anaesthesia and surgery; inhibition of anti-tumour host defences during this phase may result in the enhanced metastatic dissemination of malignant cells and the establishment of occult tumour deposits. Studies in animals tend to substantiate such a hypothesis. Many of the drugs (e.g., chemotherapeutic agents) used to treat cancer and external beam radiotherapy, have well-documented and prolonged detrimental effects on aspects of the immune response. Recently, biological immune modulators (interferons, interleukins) have been introduced into clinical practice. Recombinant technology is producing an ever-increasing variety of substances for potential use against cancer. Furthermore, also tried and tested therapies in infectious disease, such as treatments with antibiotics or antivirals, have their iatrogenic side-effects, often related to the lysis or destruction of the very micro-organism they are designed to be used against, and the release of microbe membrane fragments and/or toxins which induces additional pro-inflammatory cytokine release. For example, Norimatsu M and Morrison DC (J Infect Dis 1998 May;177(5):1302-7) found a correlation of antibiotic-induced endotoxin release and cytokine production in Escherichia coli-inoculated mouse whole blood ex vivo. Escherichia coli were incubated in mouse whole blood ex vivo supplemented with beta-lactam antibiotics that possessed preferential affinities for penicillin-binding proteins (PBPs). After 4 h, viable bacteria were undetectable in the presence of any of the 3 antibiotics tested, whereas significant increases in colony-forming units were detected in samples not treated with antibiotics. Differential levels of endotoxin in platelet-rich plasma were detected using the limulus amebocyte lysate assay, according to differential antibiotic affinities for the various PBPs. Levels of tumor necrosis factor-alpha (TNF-alpha) and interleukin-6 (IL-6) in antibiotic-treated cultures after 8 h of incubation correlated well with the levels of endotoxin at 4 h (r = .96, P < .0001 for TNF-alpha; r = .91, P = .0002 for IL-6). These data again indicate that antibiotics affect both endotoxin and cytokine responses and sometimes correlate negatively with in vivo protective efficacy of these antibiotics in gram-negative infections. Similar effects can be seen with the treatment of bacteraemia with so-called phage therapy, especially when a lytic phage is used.

Also, selective decontamination of the gut, as practiced in some patients, for example in preparation of patient for a bone marrow transplantation, induces additional pro-inflammatory cytokine release, which can add to the pro-inflammatory burst in case of a complication such as hemorrhagic shock. Also, major surgery, such as cardiopulmonary bypass predisposes the splanchnic region to inadequate perfusion and increases in gut permeability. Related to these changes, circulating endotoxin has been shown to rise during surgery, and contributes to cytokine activation, high oxygen consumption, and fever ("postperfusion syndrome"). To a large extent, free endotoxin in the gut is a product of the proliferation of aerobic gram-negative bacteria and may be reduced by nonabsorbable antibiotics, however, selective decontamination of the gut does not affect the occurrence of perioperative endotoxemia, nor does it reduce the tumor necrosis factor-alpha or interleukin-6 concentrations as determined before surgery, upon aorta declamping, 30 mins into reperfusion, or 2 hrs after surgery. Also, selective decontamination of the gut does not alter the incidence of postoperative fever or clinical outcome measures such as duration of artificial ventilation and intensive care unit and hospital stay. In conclusion, other measures are needed to affect the incidence of perioperative endotoxemia, pro-inflammatory cytokine activation and the occurrence of a postperfusion syndrome during or after surgery.

Whatever the cause may be, most iatrogenic events, herein defined as a disorder or disease resulting from a treatment of a human or animal subject with a pharmaceutical composition or by a medical or surgical procedure, resulting in the damage, destruction or lysis of cells or tissue of said subject, resulting in additional pro-inflammatory cytokine release.

The invention provides a pharmaceutical composition for the treatment of an iatrogenic event occurring in a subject, for example in a primate, and a method for the treatment of an iatrogenic event resulting in additional pro-inflammatory cytokine release, for example in a primate comprising subjecting the subject to a signalling molecule according to the invention, preferably to a mixture of such signalling molecules. Administration of such a signalling molecule or mixture preferably occurs systemically, e.g. by intravenous or intraperitoneal administration and leads to a dampening of the effect of the additionally released pro-inflammatory cytokines. In a further embodiment, such treatment also comprises the use of for example an antimicrobial agent, however, especially when such use is otherwise contraindicated or at least considered at risk because of the chance of generating toxin loads that lead to an additional pro-inflammatory cytokine response because of lysis of the microbe subject to the action of those antibiotics in an individual thus treated.

In a preferred embodiment, the invention provides a method for modulating an iatrogenic event in a subject believed to be in need thereof comprising providing said subject with a signaling molecule comprising a short, gene regulatory peptide or functional analogue thereof, wherein said signaling molecule is administered in an amount sufficient to modulate the iatrogenic event. The signal molecule is preferably a short peptide, preferably of at most 30 amino acids long, or a functional analogue or derivative thereof. In a much preferred embodiment, the peptide is an oligopeptide of from about 3 to about 15 amino acids long, preferably 4 to 12, more preferably 4 to 9, most preferably 4 to 6 amino acids long, or a functional analogue or derivative thereof. Of course, such signaling molecule can be longer, for example by extending it (N- and/or C-terminally), with more amino acids or other side groups, which can for example be (enzymatically) cleaved off when the molecule enters the place of final destination. In particular a method is provided wherein said signaling molecule modulates translocation and/or activity of a gene transcription factor. It is particularly useful when said gene transcription factor comprises an NF-kappaB/Rel protein or an AP-1 protein. Many of the iatrogenic events as mentioned above induce increased expression of inflammatory cytokines due to activation of NF-κB and/or AP-1, and in a preferred embodiment the invention provides a method wherein translocation and/or activity of said NF-kappaB/Rel protein or AP-1 protein is inhibited. In one embodiment, said peptide is selected from the group of peptides LQG, AQG, LQGV, AQGV, LQGA, VLPALP, ALPALP, VAPALP, ALPALPQ, VLPAAPQ, VLPALAQ, LAGV, VLAALP, VLAALP, VLPALA, VLPALPQ, VLAALPQ, VLPALPA, GVLPALP, LQGVLPALPQVVC, LPGCPRGVNPVVS, LPGC, MTRV, MTR, VVC. As said, additional expression of inflammatory cytokines is often due to activation of NF-κB and AP-1. Inflammatory cytokines can be expressed by endothelium (for example, by trauma), perivascular cells and adherent or transmigrating leukocytes, inducing numerous pro-inflammatory and procoagulant effects. Together these effects predispose to inflammation, thrombosis and hemorrhage. Of clinical and medical interest and value, the present invention provides the opportunity to selectively control NFκB-dependent gene expression in tissues and organs in a living subject, preferably in a primate, allowing upregulating essentially anti-inflammatory responses such as IL-10, and downregulating essentially pro-inflammatory responses such as mediated by TNF-alpha, nitric oxide (NO), IL-5, IL-6 and IL-1beta.

### Examples

The invention thus provides use of a NFκB regulating peptide or derivative thereof for the production of a pharmaceutical composition for the treatment of an iatrogenic event, preferably in a primate, and provides a method of treatment of an iatrogenic event, notably in a primate. It is preferred when the treatment comprises administering to the subject a pharmaceutical composition comprising an NFkappaB down-regulating peptide or functional analogue thereof. Examples of useful NFkappaB down-regulating peptides are VLPALPQVVC, LQGVLPALPQ, LQG, LQGV, GVLPALPQ, VLPALP, VVC, MTR and circular LQGVLPALPQVVC. More down-regulating peptides and functional analogues can be found using the methods as provided herein. Most prominent among NFkappaB down-regulating peptides are VLPALPQVVC, LQGVLPALPQ, LQG, LQGV, and VLPALP. These are also capable of reducing production of NO by a cell. It is herein also provided to use a composition that comprises at least two oligopeptides or functional analogues thereof, each capable of down-regulating NFkappaB, and thereby reducing production of NO and/or TNF-alpha by a cell, in particular wherein the at least two oligopeptides are selected from the group LQGV, AQGV and VLPALP, for the treatment of an iatrogenic event.

In another embodiment, the invention provides a mode of treatment of the additional pro-inflammatory cytokine response seen after surgical interventions. Also, selective decontamination of the gut, as practiced in some patients, for example in preparation of the patient for a bone marrow transplant, induces additional proinflammatory cytokine release, which can add to the proinflammatory burst in case of a complication such as hemorrhagic shock.

In another embodiment, the invention provides a method for clinical intervention wherein said iatrogenic event comprises destruction or lysis of a cell or tissue of said subject or of a pathogen hosted by said subject, for example wherein said lysis is due to treatment of said subject with a pharmaceutical composition. Treatment according to the invention can be achieved concomitantly with classical treatment. In response to a variety of signals received by the body as a consequence of the iatrogenic event, the NFkB/Rel family of transcription factors are activated and form different types of hetero- and homodimers among themselves to regulate the expression of target genes containing kappaB-specific binding sites. NF-kB transcription factors are hetero- or homodimers of a family of related proteins characterized by the Rel homology domain. They form two subfamilies, those containing activation domains (p65-RELA, RELB, and c-REL) and those lacking activation domains (p50, p52). The prototypical NFkB is a heterodimer of p65 (RELA) and p50 (NF-kB1) Among the activated NFkB dimers, p50-p65 heterodimers are known to be involved in enhancing the transcription of target genes and p50-p50 homodimers in transcriptional repression. However, p65-p65 homodimers are known for both transcriptional activation and repressive activity against target genes. KappaB DNA binding sites with varied affinities to different NFB dimers have been discovered in the promoters of several eukaryotic genes and the balance between activated NFkB homo- and heterodimers ultimately determines the nature and level of gene expression within the cell. The term "NFkB-regulating peptide" as used herein refers to a peptide or a modification or derivative thereof capable of modulating the activation of members of the NFkB/Rel family of transcription factors. Activation of NFkB can lead to enhanced transcription of target genes. Also, it can lead to transcriptional repression of target genes. NFkB activation can be regulated at multiple levels. For example, the dynamic shuttling of the inactive NFkB dimers between the cytoplasm and nucleus by IkappaB proteins and its termination by phosphorylation and proteasomal degradation, direct phosphorylation, acetylation of NFkB factors, and dynamic reorganization of NFkB subunits among the activated NFkB dimers have all been identified as key regulatory steps in NFkB activation and, consequently, in NFkB-mediated transcription processes. Thus, an NFkB-regulating peptide is capable of modulating the transcription of pro-inflammatory cytokine genes that are under the control of NFkB/Rel family of transcription factors. Modulating comprises the upregulation or the downregulation of transcription. In a preferred embodiment, a peptide according to the invention, or a functional derivative or analogue thereof is used for the production of a pharmaceutical composition for the treatment of iatrogenic events. NFkappaB regulating peptide can be given concomittantly, the peptide (or analogue) concentration preferably being from about 1 to about 1000mg/1, but the peptide can also been given in a bolus injection. Doses of 1 to 5 mg/kg bodyweight, for example every eight hours in a bolus injection or *per infusionem* until the patient stabilizes, are recommended. For example in cases where large adverse response to the iatrogenic event are expected or diagnosed, it is preferred to monitor cytokine profiles and arachidonic acid metabolites, such as, TNF-alpha, IL-6 or IL-10, and PGE2 and leukotrien levels, in the plasma of the treated patient, and to stop treatment according to the invention when these levels are normal. In a preferred embodiment, the invention provides a method of treating a subject suffering from an iatrogenic event with a method and signaling molecule according to the invention concomitantly, or at least timely, with a thrombolytic agent, such as (recombinant) tissue plasminogen activator, or truncated forms thereof having tissue plasminogen activity, or streptokinase, or urokinase.

The invention also provides a method for modulating an iatrogenic event in a subject that is concomitantly or already treated with a pharmaceutical composition and believed to be suffering or at risk from the side-effects of such a composition comprising providing said subject with a signaling molecule comprising a short, gene regulatory peptide or functional analogue thereof, wherein said signaling molecule is administered in an amount sufficient to modulate the side-effects, for example wherein said pharmaceutical composition is selected from the group of antigens, vaccines, antibodies, anticoagulants, antibiotics, in particular beta-lactam antibiotics, antitoxins, antibacterial agents, antiparasitic agents, antiprotozootic agents, antifungal agents, antiviral agents, cytolytic agents, cytostatic agents, thrombolytic agents, and others. The invention for example provides a method to control the toxic effects of the lysis or damage to bacterial pathogens which release endotoxin and a host of other enterotoxin and exotoxins, resulting in a at times undesirable pro-inflammatory cytokine cascade. As also shown above, the concept that controlling bacterial infections with antibiotics or lytic phages may control disease is flawed with the effect of antibiotics or phages which may kill the circulating or fixed pathogens but then at the same time release the toxins inherent to the bacterial walls or cytoplastic milieu. These antigens can induce multisystem organ disease or MODS and can trigger and be the source for the development of ARDS, SIRS, and DIC. This combines the effect of the chemokine or migratory system and the cytokine proinflammatory system which increases permeability and leakage of vascular elements and is toxic to many organ systems. Treating the patient with an NFkappaB downregulating peptide as well mitigates these effects. For this purpose, it is herein provided to provide the patient with a bolus injection of NF-kappaB down-regulating peptide such as AQGV, LQGV or VLPALP at 2mg/kg and continue an infusion with an NF-kappaB down regulating peptide such as AQGV, LQGV or VLPALP or a functional analogue thereof at a dose of 1 mg/kg bodyweight for every eight hours. Dosages may be increased or decreased, for example depending on the outcome of monitoring the cytokine profile in the plasma of the patient. In one embodiment of the present invention, a signal molecule is administered in an effective concentration to an animal or human systemically, e.g. by intravenous, intra-muscular or intraperitoneal administration. Another way of administration comprises perfusion of organs or tissue, be it *in vivo* or *ex vivo*, with a perfusion fluid comprising a signal molecule according to the invention. Bacteriophages act by killing or inactivating bacteria in a different way then most antibiotics which may already have some protective elements which lessen the likelihood of septic shock and all of its elements. Phage may change the apoptotic pathways which clear such dying or dead pathogens in such a way that these organisms are sequestered from releasing their toxins to the host. This differs dramatically from the penicillins and cephalosporin families of antibiotics which effect the destruction of bacterial cell walls or drugs such as aminoglycosides which effect protein synthesis within the bacterial cells. The penicillins are bactericidal antibiotics that impair synthesis of the bacterial cell wall peptidoglycan by attaching to penicillin-binding proteins located on the inner surface of the cell membrane. At least eight penicillin-binding proteins have been identified; they are the enzymes that are responsible for linking individual elements of the bacterial cell wall together. The cephalosporins and closely related cephamycins (e.g., cefoxitin and cefotetan) are a large and rapidly expanding group of beta-lactam antibiotics. Like the penicillins, the cephalosporins are bactericidal antibiotics that inhibit bacterial cell wall synthesis and have a low intrinsic toxicity. The adverse effects of the cephalosporins are mainly hypersensitivity reactions, local pain (with intramuscular use), and thrombophlebitis (with intravenous use). Less common toxicities include GI symptoms, elevated liver enzyme levels, and renal impairment; third- and fourth-generation cephalosporins may cause seizures, including nonconvulsive status epilepticus, in patients with renal failure. Imipenem and meropenem were the first carbapenems available for clinical use in the United States; the third, ertapenem, was released in 2002. Like other beta-lactam antibiotics, they are bactericidal and act by inhibiting bacterial cell wall synthesis. Three properties account for the extraordinarily broad antibacterial spectrum of the carbapenems: there is no permeability barrier excluding the drugs from bacteria; they have high affinity for penicillin-binding protein 2 (PBP-2), which is a crucial component of cell wall structure; and they are extremely resistant to hydrolysis by beta-lactamases. The aminoglycosides are bacterial drugs that act by binding irreversibly to the 30S ribosomal subunit of susceptible bacteria. Because oxygen is required to transport aminoglycosides across the outer bacterial membrane, these agents are ineffective against anaerobes and may function poorly in the anaerobic milieu of abscesses. Although various aminoglycosides display activity against a wide range of microorganisms, they are used chiefly to treat infections caused by aerobic gram-negative bacilli. Aminoglycosides are also used in combination with cell wall-active antibiotics (e.g., penicillins and vancomycin) for the synergistic treatment of deep tissue infections caused by enterococci and coagulase-negative staphylococci. In addition, streptomycin is still used to treat tuberculosis, tularemia, and plague. Vancomycin is a bactericidal glycopeptide that impairs cell wall synthesis of gram-positive bacteria; its spectrum of action includes staphylococci, streptococci, pneumococci, enterococci, clostridia, Corynebacterium species, and other gram-positive bacteria. It is bacteriostatic but not bactericidal against some strains of enterococci, coagulase-negative staphylococci, and corynebacteria. Quinupristin and dalfopristin are two structurally distinct streptogramins that bind to separate sites on the bacterial 50S ribosomal subunit; they thus act synergistically to inhibit protein synthesis. Although quinupristin-dalfopristin is active against a variety of bacteria, its major use is in the treatment of serious infections caused by vancomycin-resistant strains of E. faecium. The drugs may also be useful in occasional vancomycin-intolerant patients with severe infections caused by methicillin-resistant S. aureus or coagulase-negative staphylococci. Although quinupristin-dalfopristin was first marketed in 1999, resistance is already emerging. In 2000, linezolid became the first member of the oxazolidinone class to be approved for clinical use in the United States. Linezolid is a synthetic antibiotic that inhibits protein synthesis by binding to a site on the bacterial 23S ribosomal RNA of the 50S subunit, thus preventing function of the initiation complex that is required for ribosomal function. Because no other antibiotic acts in this way, bacteria that have developed resistance to other ribosomally active antimicrobials do not display cross-resistance to linezolid. Linezolid is active against nearly all aerobic gram-positive cocci at concentrations of 4 mg/ml or less, including penicillin-resistant pneumococci, methicillin-resistant staphylococci, and vancomycin-resistant enterococci; however, resistant strains have been isolated. The drug is bacteriostatic against staphylococci and enterococci, but it is bactericidal against most streptococcal strains. Linezolid is also active against L. monocytogenes, M. catarrhalis, H. influenzae, N. gonorrhoeae, B. pertussis, Pasteurella multocida, and Nocardia species. C. difficile, C. perfringens, and Bacteroides species are susceptible, but enteric gram-negative bacilli and Pseudomonas species are not. Metronidazole was first approved in 1959 for use as an antiparasitic agent; in 1981, the FDA approved an intravenous preparation of metronidazole for the treatment of serious infections caused by anaerobic bacteria. Orally administered metronidazole is excellent for treating pseudomembranous colitis caused by C. difficile. Metronidazole is also useful as part of preoperative prophylactic regimens for elective colorectal surgery. The drug is being studied for use in the treatment of nonspecific vaginitis, which is associated with Gardnerella vaginalis. Metronidazole appears to act by disrupting bacterial DNA and inhibiting nucleic acid synthesis. The drug is bactericidal against almost all anaerobic gram-negative bacilli, including B. fragilis, and against most Clostridium species. Although true anaerobic streptococci are generally susceptible to metronidazole, microaerophilic streptococci and Actinomyces and Propionibacterium species are often resistant. Metronidazole has cured a variety of infections caused by anaerobes: CNS infections, bone and joint infections, abdominal and pelvic sepsis, and endocarditis. Failures have been reported in the treatment of pleuropulmonary infections.The addition of a fluorine group and a piperazine substituent to the first quinolones has greatly improved the antibacterial spectrum of this class of drugs; the addition of a methyl group on the piperazine ring appears to further enhance the bioavailability of these compounds. There are currently 10 quinolones available for clinical use in the United States, and many additional fluoroquinolones are actively being studied. The fluoroquinolones are bactericidal compounds that act by inhibiting DNA gyrase, the bacterial enzyme responsible for maintaining the supertwisted helical structure of DNA; DNA topoisomerase IV is a secondary target. The fluoroquinolones rapidly kill bacteria, probably by impairing DNA synthesis and possibly by mechanisms involving cleaving of bacterial chromosomal DNA. Bacterial resistance to the fluoroquinolones depends on a change in their DNA gyrase. In the case of nalidixic acid, this single-step mutation occurs with a frequency of 10⁻⁷; resistance to the newer fluoroquinolones occurs much less frequently (about 10⁻ ¹¹) but is a growing concern. Bacterial strains that are resistant to one fluoroquinolone tend to be cross-resistant to related compounds; such resistance is usually mediated by chromosomes, but plasmid-mediated resistance raises the possibility of transferable resistance. The fluoroquinolones are broad-spectrum antimicrobials. Most enteric gram-negative bacilli, including E. coli, Proteus, Klebsiella, and Enterobacter, are highly susceptible; common GI pathogens, such as Salmonella, Shigella, and Campylobacter species, are also very sensitive. Other gram-negative organisms that are killed by low concentrations of the fluoroquinolones are N. gonorrhoeae and N. meningitidis, H. influenzae, P. multocida, M. catarrhalis, and Y. enterocolitica. Acinetobacter and Serratia are somewhat less susceptible. P. aeruginosa is susceptible to ciprofloxacin and trovafloxacin; ofloxacin and levofloxacin are moderately active, but the other quinolones are not effective. P. cepacia and S. maltophila are quinolone-resistant. Ciprofloxacin is the drug of choice for B. anthracis; oflaxacin and levofloxacin are also active in vitro. Among gram-positive cocci, methicillin-sensitive strains of S. aureus and coagulase-negative staphylococci are usually susceptible to quinolones, but methicillin-resistant S. aureus and enterococci are not. Lomefloxacin is not active against pneumococci and other streptococci; ciprofloxacin and ofloxacin are moderately active; and levofloxacin, sparfloxacin, gatifloxacin, moxifloxacin, and trovafloxacin are highly effective, even against non-penicillin-sensitive pneumococci. Even fastidious intracellular pathogens can be inhibited by the quinolones; Chlamydia, Mycoplasma, Listeria, Legionella, and M. tuberculosis are in this category. Only trovafloxacin is highly active against anaerobes.
Levofloxacin, gatifloxacin, moxifloxacin, and sparfloxacin demonstrate some activity against anaerobes, but the other quinolones do not. C. difficile is resistant to quinolones. Many of these families of antibiotics are walking a tightrope into the future because of resistant bacteria which can transfer their plasmids, or can be effective through the strength of their efflux pumps or their protected environments of being part of a biofilm. Both gram positive and gram negative bacteria have toxins and enzymes which cause pathogenesis. Other pathogens such as fungi and specific parasites also have these complex toxins and glycoproteins which can also mimic bacterial septic shock or an apoptotic and DIC like reaction. Most pathogens induce degrees of cytokine inflammatory response which attempt to isolate the for example, bacteria and eventually kill them but at the same time often lead to the very damage which is the hallmark of the infectious illness. This is akin to friendly fire in a war. The use of a NFkappaB down-regulating peptide in conjunction with any of the above mentioned antibiotics or in conjunction with lytic phage or even as a cocktail for concomitant use will prevent the ultimate damage unleashed by the primary infection first and then the secondary debris and release of toxins through mediation of the action of killing these bacteria with antibiotic or phage or coordination of both. Herewith, the invention provides a clinical approach where we can salvage the host even late into the disease process. This use of a peptide or functional analogue according to the invention in conjunction with antibiotics or phage turns the odds of survival of the host into a highly likely occurrence and further diminishes the endstage morbidity seen with these pathogens.

The invention also provides a method wherein said iatrogenic event includes the treatment of a subject with a virus, especially wherein lysis is due to treatment of said subject with said virus. A clear example of the beneficial use of a peptide or functional analogue thereof according to the invention to control a therapy-impeding inflammatory reaction relates to the example of an inflammatory response to (for example adenoviral or retroviral) gene vectors, e.g. in gene therapy such as in treatment of cystic fibrosis. The peptides can be administered systemically as indicated above in the case of cystic fibrosis gene therapy. In another example, said virus comprises a lytic phage used in antibacterial therapy as discussed above.

In a preferred embodiment, a peptide according to the invention, or a functional derivative or analogue thereof is used for the production of a pharmaceutical composition, for the treatment or mitigation of the additional pro-inflammatory cytokine response seen after an iatrogenic event. Examples of useful NFkappaB down-regulating peptides to be included in such a pharmaceutical composition are VLPALPQVVC, LQGVLPALPQ, LQG, LQGV, GVLPALPQ, VLPALP, VVC, MTR and circular LQGVLPALPQVVC. More gene-regulating peptides and functional analogues can be found in a (bio)assay, such as an NFkappaB translocation assay as provided herein. Most prominent among NFkappaB down-regulating peptides are VLPALPQVVC, LQGVLPALPQ, LQG, LQGV, and VLPALP. These are also capable of reducing production of NO by a cell. It is herein also provided to use a composition that comprises at least two oligopeptides or functional analogues thereof, each capable of down-regulation NFkappaB, and thereby reducing production of NO and/or TNF-alpha by a cell, in particular wherein the at least two oligopeptides are selected from the group LQGV, AQGV and VLPALP. Useful NFkappaB up-regulating peptides are VLPALPQ, GVLPALP and MTRV. As indicated, more gene-regulatory peptides may be found with an appropriate (bio)assay. A gene-regulatory peptide as used herein is preferably short. Preferably, such a peptide is 3 to 15 amino acids long, and capable of modulating the expression of a gene, such as a cytokine, in a cell. In a preferred embodiment, a peptide is a signaling molecule that is capable of traversing the plasma membrane of a cell or, in other words, a peptide that is membrane-permeable, more preferably, wherein said lead peptide is 3 to 9 amino acids long, most preferred wherein said lead peptide is 4 to 6 amino acids long.

Functional derivative or analogue herein relates to the signaling molecular effect or activity as for example can be measured by measuring nuclear translocation of a relevant transcription factor, such as NF-kappaB in an NF-kappaB assay, or AP-1 in an AP-1 assay, or by another method as provided herein. Fragments can be somewhat (i.e. 1 or 2 amino acids) smaller or larger on one or both sides, while still providing functional activity. Such a bioassay comprises an assay for obtaining information about the capacity or tendency of a peptide, or a modification thereof, to regulate expression of a gene. A scan with for example a 15-mer, or a 12-mer, or a 9-mer, or a 8-mer, or a 7-mer, or a 6-mer, or a 5-mer, or a 4-mer or a 3-mer peptide can yield valuable information on the linear stretch of amino acids that form an interaction site and allows identification of gene-regulatory peptides that have the capacity or tendency to regulate gene expression. Gene-regulatory peptides can be modified to modulate their capacity or tendency to regulate gene expression, which can be easily assayed in an in vitro bioassay such as a reporter assay. For example, some amino acid at some position can be replaced with another amino acid of similar or different properties. Alanine (Ala)-replacement scanning, involving a systematic replacement of each amino acid by an Ala residue, is a suitable approach to modify the amino acid composition of a gene-regulatory peptide when in a search for a signaling molecule capable of modulating gene expression. Of course, such replacement scanning or mapping can be undertaken with amino acids other than Ala as well, and also with D-amino acids. In one embodiment, a peptide derived from a naturally occurring polypeptide is identified as being capable of modulating gene expression of a gene in a cell. Subsequently, various synthetic Ala-mutants of this gene-regulatory peptide are produced. These Ala-mutants are screened for their enhanced or improved capacity to regulate expression of a gene compared to gene-regulatory polypeptide.

Furthermore, a gene-regulatory peptide, or a modification or analogue thereof, can be chemically synthesised using D- and / or L-stereoisomers. For example, a gene-regulatory peptide that is a retro-inverso of an oligopeptide of natural origin is produced. The concept of polypeptide retro-inversion (assemblage of a natural L-amino acid-containing parent sequence in reverse order using D-amino acids) has been applied successfully to synthetic peptides. Retro-inverso modification of peptide bonds has evolved into a widely used peptidomimetic approach for the design of novel bioactive molecules which has been applied to many families of biologically active peptides. The sequence, amino acid composition and length of a peptide will influence whether correct assembly and purification are feasible. These factors also determine the solubility of the final product. The purity of a crude peptide typically decreases as the length increases. The yield of peptide for sequences less than 15 residues is usually satisfactory, and such peptides can typically be made without difficulty. The overall amino acid composition of a peptide is an important design variable. A peptide's solubility is strongly influenced by composition. Peptides with a high content of hydrophobic residues, such as Leu, Val, Ile, Met, Phe and Trp, will either have limited solubility in aqueous solution or be completely insoluble. Under these conditions, it can be difficult to use the peptide in experiments, and it may be difficult to purify the peptide if necessary. To achieve a good solubility, it is advisable to keep the hydrophobic amino acid content below 50% and to make sure that there is at least one charged residue for every five amino acids. At physiological pH Asp, Glu, Lys, and Arg all have charged side chains. A single conservative replacement, such as replacing Ala with Gly, or adding a set of polar residues to the N- or C-terminus, may also improve solubility. Peptides containing multiple Cys, Met, or Trp residues can also be difficult to obtain in high purity partly because these residues are susceptible to oxidation and/or side reactions. If possible, one should choose sequences to minimize these residues. Alternatively, conservative replacements can be made for some residues. For instance, Norleucine can be used as a replacement for Met, and Ser is sometimes used as a less reactive replacement for Cys. If a number of sequential or overlapping peptides from a protein sequence are to be made, making a change in the starting point of each peptide may create a better balance between hydrophilic and hydrophobic residues. A change in the number of Cys, Met, and Trp residues contained in individual peptides may produce a similar effect. In another embodiment of the invention, a gene-regulatory peptide capable of modulating gene expression is a chemically modified peptide. A peptide modification includes phosphorylation (e.g on a Tyr, Ser or Thr residue), N-terminal acetylation, C-terminal amidation, C-terminal hydrazide, C-terminal methyl ester, fatty acid attachment, sulfonation (tyrosine), N-terminal dansylation, N-terminal succinylation, tripalmitoyl-S-Glyceryl Cysteine (PAM3 Cys-OH) as well as farnesylation of a Cys residue. Systematic chemical modification of a gene-regulatory peptide can for example be performed in the process of gene-regulatory peptide optimalization.

Synthetic peptides can be obtained using various procedures known in the art. These include solid phase peptide synthesis (SPPS) and solution phase organic synthesis (SPOS) technologies. SPPS is a quick and easy approach to synthesize peptides and small proteins. The C-terminal amino acid is typically attached to a cross-linked polystyrene resin via an acid labile bond with a linker molecule. This resin is insoluble in the solvents used for synthesis, making it relatively simple and fast to wash away excess reagents and by-products.

The peptides as mentioned in this document such as LQG, AQG, LQGV, AQGV, LQGA, VLPALP, ALPALP, VAPALP, ALPALPQ, VLPAAPQ, VLPALAQ, LAGV, VLAALP, VLPALA, VLPALPQ, VLAALPQ, VLPALPA, GVLPALP, VVCNYRDVRFESIRLPGCPRGVNPVVSYAVALSCQCAL, RPRCRPINATLAVEKEGCPVCITVNTTICAGYCPT, SKAPPPSLPSPSRLPGPS, LQGVLPALPQVVC, SIRLPGCPRGVNPVVS, LPGCPRGVNPVVS, LPGC, MTRV, MTR, and VVC were prepared by solid-phase synthesis using the fluorenylmethoxycarbonyl (Fmoc)/tert-butyl-based methodology with 2-chlorotrityl chloride resin as the solid support. The side-chain of glutamine was protected with a trityl function. The peptides were synthesized manually. Each coupling consisted of the following steps: (i) removal of the alpha-amino Fmoc-protection by piperidine in dimethylformamide (DMF), (ii) coupling of the Fmoc amino acid (3 eq) with diisopropylcarbodiimide (DIC)/1-hydroxybenzotriazole (HOBt) in DMF/N-methylformamide (NMP) and (iii) capping of the remaining amino functions with acetic anhydride/diisopropylethylamine (DIEA) in DMF/NMP. Upon completion of the synthesis, the peptide resin was treated with a mixture of trifluoroacetic acid (TFA)/H₂O/triisopropylsilane (TIS) 95:2.5:2.5. After 30 minutes TIS was added until decolorization. The solution was evaporated in vacuo and the peptide precipitated with diethylether. The crude peptides were dissolved in water (50-100 mg/ml) and purified by reverse-phase high-performance liquid chromatography (RP-HPLC). HPLC conditions were: column: Vydac TP21810C18 (10 x 250 mm); elution system: gradient system of 0.1% TFA in water v/v (A) and 0.1% TFA in acetonitrile (ACN) v/v (B); flow rate 6 ml/min; absorbance was detected from 190-370 nm. There were different gradient systems used. For example for peptides LQG and LQGV: 10 minutes 100% A followed by linear gradient 0-10% B in 50 minutes. For example for peptides VLPALP and VLPALPQ: 5 minutes 5% B followed by linear gradient 1% B/minute. The collected fractions were concentrated to about 5 ml by rotation film evaporation under reduced pressure at 40°C. The remaining TFA was exchanged against acetate by eluting two times over a column with anion exchange resin (Merck II) in acetate form. The elute was concentrated and lyophilised in 28 hours. Peptides later were prepared for use by dissolving them in PBS.

RAW 264.7 macrophages, obtained from American Type Culture Collection (Manassas, VA), were cultured at 37°C in 5% CO2 using DMEM containing 10% FBS and antibiotics (100 U/ml of penicillin, and 100 µg/ml streptomycin). Cells (1 x10⁶/ml) were incubated with peptide (10 µg/ml) in a volume of 2 ml. After 8 h of culture cells were washed and prepared for nuclear extracts.

Nuclear extracts and EMSA were prepared according to Schreiber et al. Methods (Schrieber et al. 1989, Nucleic Acids Research 17). Briefly, nuclear extracts from peptide stimulated or nonstimulated macrophages were prepared by cell lysis followed by nuclear lysis. Cells were then suspended in 400 µl of buffer (10 mM HEPES (pH 7.9), 10 mM KCl, 0.1 mM KCL, 0.1 mM EDTA, 0.1 mM EGTA, 1 mM DTT, 0.5 mM PMSF and protease inhibitors), vigorously vortexed for 15 s, left standing at 4°C for 15 min, and centrifuged at 15,000 rpm for 2 min. The pelleted nuclei were resuspended in buffer (20 mM HEPES (pH 7.9), 10% glycerol, 400 mM NaCl, 1 mM EDTA, 1mM EGTA, 1 mM DTT, 0.5 mM PMSF and protease inhibitors) for 30 min on ice, then the lysates were centrifuged at 15,000 rpm for 2 min. The supernatants containing the solubilized nuclear proteins were stored at -70°C until used for the Electrophoretic Mobility Shift Assays (EMSA).

Electrophoretic mobility shift assays were performed by incubating nuclear extracts prepared from control (RAW 264.7) and peptide treated RAW 264.7 cells with a 32P-labeled double-stranded probe (5' AGCTCAGAGGGGGACTTTCCGAGAG 3') synthesized to represent the NF-kappaB binding sequence. Shortly, the probe was end-labeled with T4 polynucleotide kinase according to manufacturer's instructions (Promega, Madison, WI). The annealed probe was incubated with nuclear extract as follows: in EMSA, binding reaction mixtures (20 µl) contained 0.25 µg of poly(dI-dC) (Amersham Pharmacia Biotech) and 20,000 rpm of 32P-labeled DNA probe in binding buffer consisting of 5 mM EDTA, 20% Ficoll, 5 mM DTT, 300 mM KCl and 50 mM HEPES. The binding reaction was started by the addition of cell extracts (10 µg) and was continued for 30 min at room temperature. The DNA-protein complex was resolved from free oligonucleotide by electrophoresis in a 6% polyacrylamide gel. The gels were dried and exposed to x-ray films.

The transcription factor NF-kB participates in the transcriptional regulation of a variety of genes. Nuclear protein extracts were prepared from LPS and peptide treated RAW264.7 cells or from LPS treated RAW264.7 cells. In order to determine whether the peptide modulates the translocation of NF-kB into the nucleus, on these extracts EMSA was performed. Here we see that indeed some peptides are able to modulate the translocation of NF-kB since the amount of labeled oligonucleotide for NF-kB is reduced. In this experiment peptides that show the modulation of translocation of NF-kB are: VLPALPQVVC, LQGVLPALPQ, LQG, LQGV, GVLPALPQ, VLPALP, VLPALPQ, GVLPALP, VVC, MTRV, MTR.

RAW 264.7 mouse macrophages were cultured in DMEM, containing 10% or 2% FBS, penicillin, streptomycin and glutamine, at 37 °C, 5% CO₂. Cells were seeded in a 12-wells plate (3x10⁶ cells/ml) in a total volume of 1 ml for 2hours and then stimulated with LPS (E. coli 026:B6; Difco Laboratories, Detroit, MI, USA) and/or peptide (1 microgr/ml). After 30 minutes of incubation plates were centrifuged and cells were collected for nuclear extracts. Nuclear extracts and EMSA were prepared according to Schreiber et al. Cells were collected in a tube and centrifuged for 5 minutes at 2000 rpm (rounds per minute) at 4°C (Universal 30 RF, Hettich Zentrifuges). The pellet was washed with ice-cold Tris buffered saline (TBS pH 7.4) and resuspended in 400 µl of a hypotonic buffer A (10 mM HEPES pH 7.9, 10 mM KCl, 0.1 mM EDTA, 0.1 mM EGTA, 1 mM DTT, 0.5 mM PMSF and protease inhibitor cocktail (Complete^{TM} Mini, Roche) and left on ice for 15 minutes. Twenty five micro litre 10% NP-40 was added and the sample was centrifuged (2 minutes, 4000 rpm, 4°C). The supernatant (cytoplasmic fraction) was collected and stored at -70°C. The pellet, which contains the nuclei, was washed with 50 µl buffer A and resuspended in 50 µl buffer C (20 mM HEPES pH 7.9, 400 mM NaCl, 1 mM EDTA, 1 mM EGTA, 1 mM DTT, 0.5 mM PMSF and protease inhibitor cocktail and 10% glycerol). The samples were left to shake at 4°C for at least 60 minutes. Finally the samples were centrifuged and the supernatant (nucleic fraction) was stored at -70°C.

Bradford reagent (Sigma) was used to determine the final protein concentration in the extracts. For Electrophoretic mobility shift assays an oligonucleotide representing NF-κB binding sequence (5'-AGC TCA GAG GGG GAC TTT CCG AGA G-3') was synthesized. Hundred pico mol sense and antisense oligo were annealed and labelled with γ-³²P-dATP using T4 polynucleotide kinase according to the manufacture's instructions (Promega, Madison, WI). Nuclear extract (5-7.5 µg) was incubated for 30 minutes with 75000 cpm probe in binding reaction mixture (20 microliter) containing 0.5 µg poly dI-dC (Amersham Pharmacia Biotech) and binding buffer BSB (25 mM MgCl₂, 5 mM CaCl₂, 5mM DTT and 20% Ficoll) at room temperature. The DNA-protein complex was resolved from free oligonucleotide by electrophoresis in a 4-6% polyacrylamide gel (150 V, 2-4 hours). The gel was then dried and exposed to x-ray film. The transcription factor NF-kB participates in the transcriptional regulation of a variety of genes. Nuclear protein extracts were prepared from either LPS (1 mg/ml), peptide (1 mg/ml) or LPS in combination with peptide treated and untreated RAW264.7 cells. In order to determine whether the peptides modulate the translocation of NF-kB into the nucleus, on these extracts EMSA was performed. Peptides are able to modulate the basal as well as LPS induced levels of NF-kB. In this experiment peptides that show the inhibition of LPS induced translocation of NF-kB are: VLPALPQVVC, LQGVLPALPQ, LQG, LQGV, GVLPALPQ, VLPALP, VVC, MTR and circular LQGVLPALPQVVC. Peptides that in this experiment promote LPS induced translocation of NF-kB are: VLPALPQ, GVLPALP and MTRV. Basal levels of NF-kB in the nucleus were decreased by VLPALPQVVC, LQGVLPALPQ, LQG and LQGV while basal levels of NF-kB in the nucleus were increased by GVLPALPQ, VLPALPQ, GVLPALP, VVC, MTRV, MTR and LQGVLPALPQVVC. In other experiments, QVVC also showed the modulation of translocation of NF-kB into nucleus (data not shown). Further modes of identification of gene-regulatory peptides by NFkB analysis
*Cells:* Cells will be cultured in appropriate culture medium at 37 °C, 5% CO₂. Cells will be seeded in a 12-wells plate (usually 1x10⁶ cells/ml) in a total volume of 1 ml for 2hours and then stimulated with regulatory peptide in the presence or absence of additional stimuli such as LPS. After 30 minutes of incubation plates will be centrifuged and cells are collected for cytosolic or nuclear extracts.
*Nuclear Extracts:* Nuclear extracts and EMSA could be prepared according to Schreiber et al. Method (Schriber et al. 1989, Nucleic Acids Research 17). Cells are collected in a tube and centrifuged for 5 minutes at 2000 rpm (rounds per minute) at 4°C (Universal 30 RF, Hettich Zentrifuges). The pellet is washed with ice-cold Tris buffered saline (TBS pH 7.4) and resuspended in 400 µl of a hypotonic buffer A (10 mM HEPES pH 7.9, 10 mM KCl, 0.1 mM EDTA, 0.1 mM EGTA, 1 mM DTT, 0.5 mM PMSF and protease inhibitor cocktail (Complete^{TM} Mini, Roche) and left on ice for 15 minutes. Twenty five micro litre 10% NP-40 is added and the sample is centrifuged (2 minutes, 4000 rpm, 4°C). The supernatant (cytoplasmic fraction) was collected and stored at -70°C for analysis. The pellet, which contains the nuclei, is washed with 50 µl buffer A and resuspended in 50 µl buffer C (20 mM HEPES pH 7.9, 400 mM NaCl, 1 mM EDTA, 1 mM EGTA, 1 mM DTT, 0.5 mM PMSF and protease inhibitor cocktail and 10% glycerol). The samples are left to shake at 4°C for at least 60 minutes. Finally the samples are centrifuged and the supernatant (nucleic fraction) is stored at -70°C for analysis.
Bradford reagent (Sigma) could be used to determine the final protein concentration in the extracts.
*EMSA:* For Electrophoretic mobility shift assays an oligonucleotide representing NF-κB binding sequence such as (5'-AGC TCA GAG GGG GAC TTT CCG AGA G-3') are synthesized. Hundred pico mol sense and antisense oligo are annealed and labeled with γ-³²P-dATP using T4 polynucleotide kinase according to manufacture's instructions (Promega, Madison, WI). Cytosolic extract or nuclear extract (5-7.5 µg) from cells treated with regulatory peptide or from untreated cells is incubated for 30 minutes with 75000 cpm probe in binding reaction mixture (20 □l) containing 0.5 µg poly dI-dC (Amersham Pharmacia Biotech) and binding buffer BSB (25 mM MgCl₂, 5 mM CaCl₂, 5mM DTT and 20% Ficoll) at room temperature. Or cytosolic and nuclear extract from untreated cells or from cells treated with stimuli could also be incubated with probe in binding reaction mixture and binding buffer. The DNA-protein complex are resolved from free oligonucleotide by electrophoresis in a 4-6% polyacrylamide gel (150 V, 2-4 hours). The gel is then dried and exposed to x-ray film. Peptides can be biotinylated and incubated with cells. Cells are then washed with phosphate-buffered saline, harvested in the absence or presence of certain stimulus (LPS, PHA, TPA, anti-CD3, VEGF, TSST-1, VIP or know drugs etc.). After culturing cells are lysed and cells lysates (whole lysate, cytosolic fraction or nuclear fraction) containing 200 micro gram of protein are incubated with 50 miroliter Neutr-Avidin-plus beads for 1 h at 4°C with constant shaking. Beads are washed five times with lysis buffer by centrifugation at 6000 rpm for 1 min. Proteins are eluted by incubating the beads in 0.05 N NaoH for 1 min at room temperature to hydrolyze the protein-peptide linkage and analyzed by SDS-polyacrylamide gel electrophoresis followed by immunoprecipitated with agarose-conjugated anti-NF-kB subunits antibody or immunoprecipitated with antibody against to be studied target. After hydrolyzing the protein-peptide linkage, the sample could be analyzed on HPLS and mass-spectrometry. Purified NF-kB subunits or cell lysate interaction with biotinylated regulatory peptide can be analysed on biosensor technology. Peptides can be labeled with FITC and incubated with cells in the absence or presence of different stimulus. After culturing, cells can be analysed with fluorescent microscopy, confocal microscopy, flow cytometry (cell membrane staining and/or intracellular staining) or cells lysates are made and analysed on HPLC and mass-spectrometry. NF-kB transfected (reporter gene assay) cells and gene array technology can be used to determine the regulatory effects of peptides.
*HPLC and mass-spectrometry analysis:* Purified NF-kB subunit or cytosolic/nuclear extract is incubated in the absence or presence of (regulatory) peptide is diluted (2:1) with 8 N guanidinium chloride and 0.1% trifluoracetic acid, injected into a reverse-phase HPLC column (Vydac C18) equilibrated with solvent A (0.1% trifluroacetic acid), and eluted with a gradient of 0 to 100% eluant B (90% acetonitrile in solvent A). Factions containing NF-kB subunit are pooled and concentrated. Fractions are then dissolved in appropriate volume and could be analysed on mass-spectrometry.

### Further references:

WO99/59617, WO01/72831 WO97/49721, WO01/10907, WO01/11048.

## Claims

1. A method for modulating an iatrogenic event in a subject comprising providing said subject with a gene-regulatory peptide or functional analogue thereof.

2. A method according to claim 1 wherein said peptide or analogue modulates translocation and/or activity of a gene transcription factor.

3. A method according to claim 1 or 2 wherein said gene transcription factor comprises an NF-kappaB/Rel protein.

4. A method according to claim 1, 2 or 3 wherein translocation and/or activity of said NF-kappaB/Rel protein is inhibited.

5. A method according to anyone of claims 1 to 4 wherein said iatrogenic event comprises destruction or lysis of a cell or tissue of said subject or of a pathogen hosted by said subject.

6. A method according to claim 5 wherein said lysis is due to treatment of said subject with a pharmaceutical composition.

7. A method according to claim 6 wherein said pharmaceutical composition is selected from the group of antigens, vaccines, antibodies, anticoagulants, antibiotics, antitoxins, antibacterial agents, antiparasitic agents, antiprotozootic agents, antifungal agents, antiviral agents, cytolytic agents, cytostatic agents, thrombolytic agents

8. A method according to claim 5 wherein said lysis is due to treatment of said subject with a virus.

9. A method according to claim 8 wherein said virus comprises a lytic phage.

10. Use of a signaling molecule comprising a NF-kappaB down-regulating peptide or functional analogue thereof for the production of a pharmaceutical composition for the treatment of a pro-inflammatory cytokine response occurring after an iatrogenic event in a subject.

11. Use according to claim 10 wherein translocation and/or activity of said NF-kappaB/Rel protein is inhibited.

12. Use according to claim 10 or 11 wherein said iatrogenic event comprises destruction or lysis of a cell or tissue of said subject or of a pathogen hosted by said subject.

13. Use according to claim 12 wherein said lysis is due to treatment of said subject with a pharmaceutical composition.
